# EUROPEAN PATENT APPLICATION

(11) **EP 4 488 359 A1**
(43) Date of publication of application: **08.01.2025**
(21) Application number: 23784777.7
(22) Date of filing: 05.04.2023
(51) Int. Cl.: C12M 1/34, G02B 21/34, G02B 21/36

(54) **ASSESSMENT SYSTEM, INFORMATION PROCESSING DEVICE, AND INFORMATION PROCESSING METHOD**

(30) Priority: 06.04.2022 JP 2022063642
(71) Applicant: FUJIFILM Corporation, Tokyo 106-8620 (JP)
(72) Inventor: SHIRAISHI, Yasushi, Ashigarakami-gun, Kanagawa 258-8577 (JP)
(74) Representative: Hoffmann Eitle
(86) International application number: PCT/JP2023/014108
(87) International publication number: WO 2023/195493

(57) **Abstract**

An evaluation system of the present disclosure is an evaluation system that evaluates a seeding state of cells seeded in a cell culture vessel having at least one electrode disposed on a bottom surface, the evaluation system including a microscope device that generates image data by imaging the electrode and the cells from a surface side of the electrode of the cell culture vessel, and an information processing device that analyzes the image data and determines whether the seeding state is appropriate based on an analysis result.

## Description

### BACKGROUND OF THE INVENTION

### 1. Field of the Invention

The technology of the present disclosure relates to an evaluation system, an information processing device, and an information processing method.

### 2. Description of the Related Art

In order to improve an efficiency of new drug development, for example, a method of performing a toxicity evaluation using cells such as myocardial cells produced from induced pluripotent stem (iPS) cells has been developed (see, for example, WO2019/131806A). The toxicity evaluation is performed by evaluating responsiveness of cells to a drug.

As a culture vessel for the cells, for example, a well plate in which a plurality of wells are formed is used. A microelectrode array (MEA) in which a plurality of minute measurement electrodes are disposed is provided on a bottom surface of each well. Such a well plate is called an MEA plate. A waveform (for example, a myocardial waveform indicating pulsation of the myocardial cells) indicating an electrophysiological change of cells cultured in the wells is output from each measurement electrode of the microelectrode array. The toxicity evaluation is performed by measuring a change in waveform with respect to a drug.

Variations occur in the waveform output from each of the plurality of measurement electrodes provided in the wells. Therefore, for each well, one measurement electrode from which the most ideal waveform is output is selected as a target electrode to be a target for the toxicity evaluation. Such selection of the target electrode is also called selection of the golden channel. For example, in a case of the myocardial cells, a waveform closest to the healthiest state is selected among the myocardial waveforms output from the measurement electrodes based on a known myocardial waveform representing a healthy state (for example, a state without a disease such as arrhythmia) obtained in the past measurement.

### SUMMARY OF THE INVENTION

However, the measurement electrode (that is, the golden channel) selected as the target electrode is merely an electrode that is relatively suitable for the toxicity evaluation among a plurality of measurement electrodes whose waveforms are measured. That is, this does not guarantee that a waveform output from the golden channel is truly measured correctly. For example, in a case in which the cells are not appropriately seeded in the well, the waveform is not suitable for the toxicity evaluation, resulting in reduced evaluation accuracy.

An object of the technology of the present disclosure is to provide an evaluation system, an information processing device, and an information processing method that enable improvement in evaluation accuracy of a toxicity evaluation.

In order to achieve the above object, an evaluation system of the present disclosure is an evaluation system that evaluates a seeding state of cells seeded in a cell culture vessel having at least one electrode disposed on a bottom surface, the evaluation system comprising: a microscope device that generates image data by imaging the electrode and the cells from a surface side of the electrode of the cell culture vessel; and an information processing device that analyzes the image data and determines whether the seeding state is appropriate based on an analysis result.

It is preferable that the microscope device is an epifluorescence microscope that generates the image data by irradiating the cells stained with a fluorescent dye with excitation light and capturing an image of fluorescence emitted from the cells.

It is preferable that the microscope device generates the image data for specifying a region of the cells and a region of the electrode by capturing an image of the excitation light reflected by the electrode in addition to the fluorescence.

It is preferable that the microscope device generates the image data for specifying a region of the cells and a region of the electrode by irradiating the cells and the electrode with illumination light and capturing an image of reflected light from the cells and the electrode.

It is preferable that the electrode includes a measurement electrode for measuring a potential and at least one of a reference electrode to which a reference potential is applied or a stimulation electrode for applying a stimulus to the cells.

It is preferable that the cells are smaller than a size of the measurement electrode, and a plurality of the cells are seeded on the measurement electrode.

It is preferable that the plurality of seeded cells exhibit a single behavior.

It is preferable that the cells are myocardial cells.

It is preferable that the information processing device calculates a cell coverage on the reference electrode or on the stimulation electrode by analyzing the image data, and determines that the seeding state is appropriate in a case in which the calculated cell coverage is equal to or less than a threshold value.

It is preferable that in a case in which the cell coverage is not equal to or less than the threshold value, the information processing device estimates an influence on a measurement waveform based on the cell coverage.

It is preferable that the information processing device analyzes a state of the cells on the measurement electrode based on the image data.

It is preferable that the information processing device issues a warning in a case in which the seeding state is not appropriate.

An information processing device of the present disclosure is an information processing device that evaluates a seeding state of cells seeded in a cell culture vessel having at least one electrode disposed on a bottom surface, the information processing device comprising: a processor, in which the processor acquires image data generated by imaging the electrode and the cells from a surface side of the electrode of the cell culture vessel with a microscope device, and analyzes the image data and determines whether the seeding state is appropriate based on an analysis result.

An information processing method of the present disclosure is an information processing method of evaluating a seeding state of cells seeded in a cell culture vessel having at least one electrode disposed on a bottom surface, the information processing method comprising: acquiring image data generated by imaging the electrode and the cells from a surface side of the electrode of the cell culture vessel with a microscope device; and analyzing the image data and determining whether the seeding state is appropriate based on an analysis result.

According to the technology of the present disclosure, it is possible to provide an evaluation system, an information processing device, and an information processing method that enable improvement in evaluation accuracy of a toxicity evaluation.

### BRIEF DESCRIPTION OF THE DRAWINGS

Fig. 1 is a diagram schematically showing an electrical function evaluation system.
Fig. 2 is a perspective view showing an example of an MEA plate.
Fig. 3 is a diagram showing an example of a well.
Fig. 4 is a diagram showing a configuration example of a bottom surface of the well.
Fig. 5 is a block diagram showing an example of a hardware configuration of an evaluation system.
Fig. 6 is a diagram showing an example of a myocardial waveform.
Fig. 7 is a diagram showing an example of waveforms output from 16 electrodes included in a microelectrode array.
Fig. 8 is a diagram schematically showing an example of a configuration of a microscope device.
Fig. 9 is a diagram showing an example of a relationship between a seeding state of a cell group and a measurement waveform.
Fig. 10 is a diagram showing an example of a relationship between a seeding state of the cell group and a measurement waveform.
Fig. 11 is a graph showing a relationship between a cell coverage on a reference electrode and an offset.
Fig. 12 is a diagram showing an example of an equivalent circuit of a model composed of an electrode and cells during waveform measurement.
Fig. 13 is a block diagram showing an example of a functional configuration of an information processing device.
Fig. 14 is a diagram conceptually showing an example of image analysis by an image analysis unit.
Fig. 15 is a flowchart showing an example of processing by the image analysis unit and a determination unit.
Fig. 16 is a diagram showing an example of an evaluation flow.
Fig. 17 is a diagram showing an example of a measurement waveform in a case in which cells are laminated on an electrode.

### DESCRIPTION OF THE PREFERRED EMBODIMENTS

Hereinafter, an embodiment according to the technology of the present disclosure will be described with reference to the drawings.

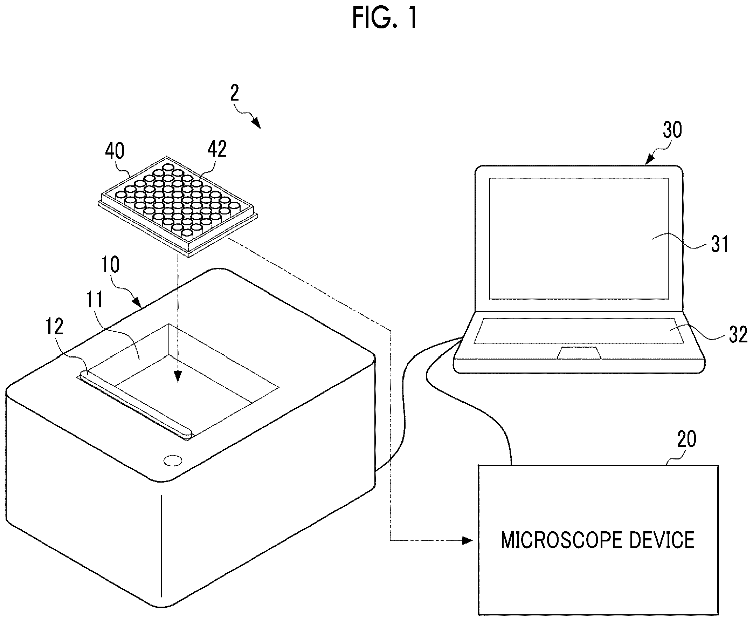

Fig. 1 schematically shows an evaluation system 2 that measures electrical activity of cells. The evaluation system 2 shown in Fig. 1 is configured of a potential measurement device 10, a microscope device 20, and an information processing device 30. The potential measurement device 10 measures a waveform (for example, a myocardial waveform indicating pulsation of myocardial cells) indicating an electrophysiological change in a cell group cultured by a culture device (not shown).

An MEA plate 40 is used for culturing the cells. The potential measurement device 10 is provided with an accommodation part 11 that accommodates the MEA plate 40 therein. In addition, the potential measurement device 10 is provided with a slide-type lid 12 for opening and closing the accommodation part 11. The MEA plate 40 is accommodated in the accommodation part 11 with the cells seeded thereon.

In the present embodiment, myocardial cells produced from iPS cells are used as the cells. In addition, the potential measurement device 10 measures an extracellular potential representing a myocardial waveform of the myocardial cells seeded on the MEA plate 40 by a multipoint measurement method, and outputs waveform data obtained by the measurement to the information processing device 30. The waveform data represents a pulse signal output by the myocardial cells.

The microscope device 20 generates image data by imaging a plurality of culture wells (hereinafter, simply referred to as wells) 42 formed in the MEA plate 40, on which the waveform is measured by the potential measurement device 10, and outputs the generated image data to the information processing device 30.

The information processing device 30 is configured of a general computer such as a personal computer. The information processing device 30 is installed with software for analyzing the waveform data input from the potential measurement device 10 and for analyzing the image data input from the microscope device 20.

The information processing device 30 includes a display unit 31 and an input unit 32. The display unit 31 is a display device such as a liquid crystal display or an organic electro luminescence (EL) display. The input unit 32 is an input device such as a keyboard, a touch pad, or a mouse. The information processing device 30 is connected to the potential measurement device 10 and the microscope device 20 via a wired or wireless connection. The display unit 31 and the input unit 32 may be configured as external devices connected to the information processing device 30.

The information processing device 30 calculates an extracellular potential duration (field potential duration (FPD)), an interspike interval (ISI), and the like based on the input waveform data. Since the FPD corresponds to a QT interval (time from a start of a Q wave to an end of a T wave) in an electrocardiogram, it is used as an index of arrhythmia. Prolongation of the QT interval indicates a potential for arrhythmia. It is possible to perform a toxicity evaluation for evaluating responsiveness of the cells to a drug candidate compound, based on the FPD or the like.

In addition, the information processing device 30 analyzes the image data input from the microscope device 20 and determines whether a seeding state of the cells is appropriate based on an analysis result.

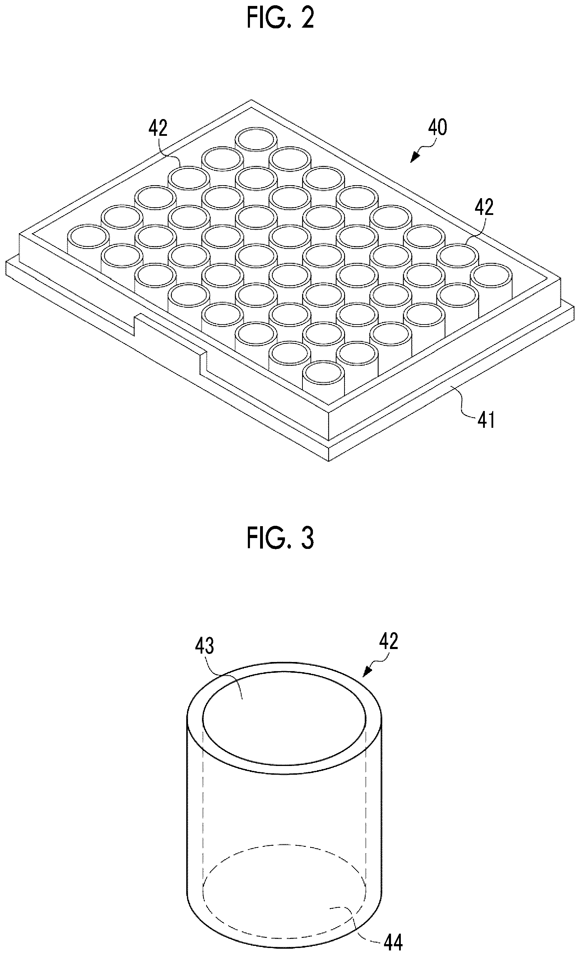

Fig. 2 shows an example of the MEA plate 40. The MEA plate 40 is a multi-well plate in which a plurality of wells 42 are arranged on a substrate 41. The MEA plate 40 shown in Fig. 2 has 48 wells 42. The number of the wells 42 provided in the MEA plate 40 is not limited to 48, and may be 24, 96, or the like.

Fig. 3 shows an example of the well 42. The well 42 is a substantially cylindrical vessel with an opening 43 formed at an upper part. The plurality of cells are seeded by dropping a cell suspension onto a bottom surface 44 of the well 42. The myocardial cells are adherent cells and are grown by adhering to the bottom surface 44 that serves as a cell adhesion surface. The plurality of seeded myocardial cells are bonded to each other to form a cell group (so-called myocardial sheet) exhibiting a single behavior (that is, pulsation). In addition, a culture solution containing a culture medium is injected into the well 42. The well 42 is an example of a "cell culture vessel" according to the technology of the present disclosure.

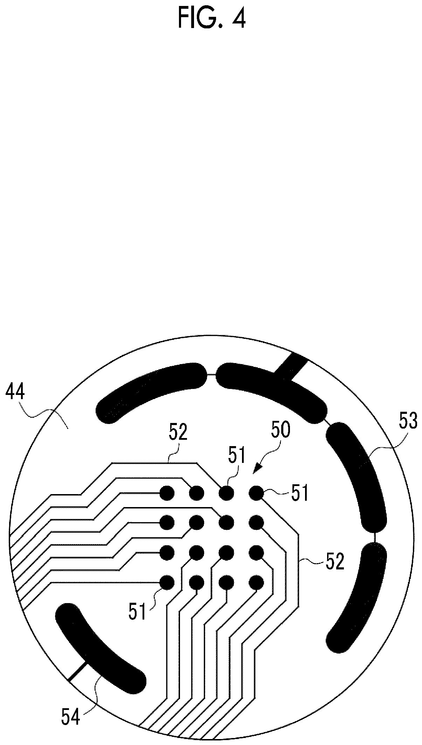

Fig. 4 shows a configuration example of the bottom surface 44 of the well 42. A microelectrode array 50 is embedded in the bottom surface 44 of the well 42. The microelectrode array 50 has a plurality of measurement electrodes 51 for potential measurement. In the example shown in Fig. 4, the microelectrode array 50 has 16 measurement electrodes 51 arranged in a 4 × 4 square pattern. The measurement electrode 51 is exposed on the bottom surface 44 of the well 42 and comes into contact with the seeded cells. Each of the measurement electrodes 51 is connected to a potential measurement circuit 60, which will be described below, via a wiring line 52. Hereinafter, the measurement electrode 51 may be denoted by a channel CH. In addition, the 16 measurement electrodes 51 are distinguished by being denoted by channels CH1 to CH16.

In addition to the microelectrode array 50, a reference electrode 53 is provided on the bottom surface 44 of the well 42. The reference electrode 53 is disposed around the microelectrode array 50 along an outer edge of the bottom surface 44 so as not to come into contact with the cells to be measured. A ground potential as a reference potential is applied to the reference electrode 53. The extracellular potential is measured from a potential difference between the measurement electrode 51 and the reference electrode 53. One cell to be seeded is smaller than a size of the measurement electrode 51. A plurality of cells (approximately 2 to 3 cells) are seeded on one measurement electrode 51.

In addition, a stimulation electrode 54 for applying a stimulus to the cells is provided on the bottom surface 44 of the well 42. Note that it is not essential to apply a stimulus to the cells, and the stimulation electrode 54 does not have to be provided in the well 42.

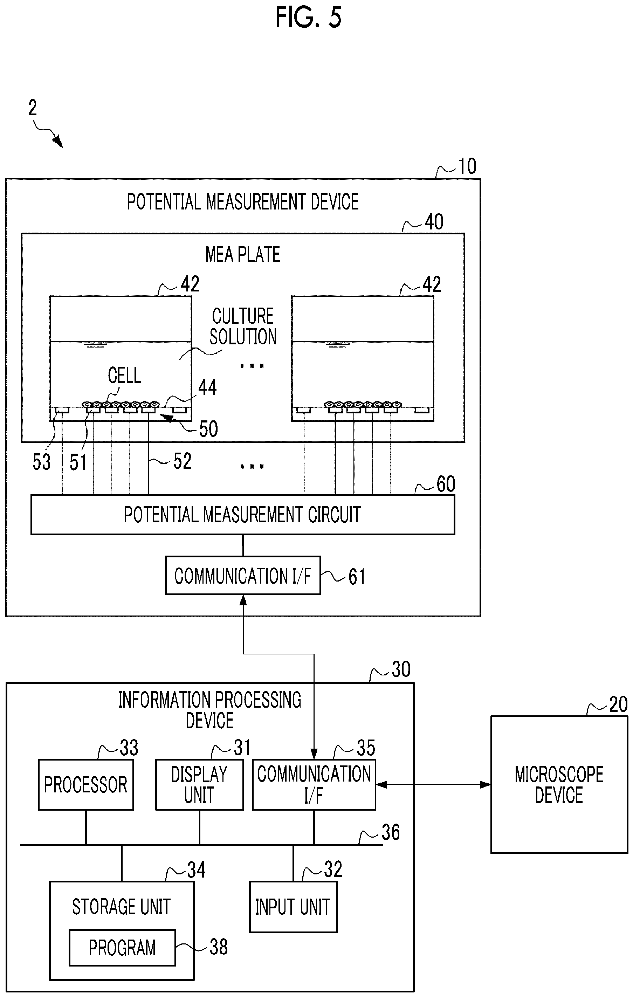

Fig. 5 shows an example of a hardware configuration of the evaluation system 2. The potential measurement device 10 includes the potential measurement circuit 60 and a communication interface (I/F) 61. The potential measurement circuit 60 measures the extracellular potential of the cell group in the well 42 of the MEA plate 40. Specifically, the potential measurement circuit 60 measures the extracellular potential via each measurement electrode 51 of the microelectrode array 50 provided in each of the wells 42. That is, the potential measurement circuit 60 measures 16 waveforms for each well 42. The potential measurement circuit 60 outputs the measured waveform to the information processing device 30 as waveform data via the communication I/F 61.

The information processing device 30 includes a processor 33, a storage unit 34, the input unit 32, the display unit 31, a communication I/F 35, and a bus 36. The processor 33 is a computer that realizes various functions by reading out a program 38 and various kinds of data stored in the storage unit 34 and executing processing. The processor 33 is, for example, a central processing unit (CPU).

The storage unit 34 is a storage device that stores the program 38 and the various kinds of data in a case in which the processor 33 executes processing. The storage unit 34 includes, for example, a random access memory (RAM), a read only memory (ROM), or a storage. The RAM is, for example, a volatile memory used as a work area or the like of the processor 33. The ROM is, for example, a non-volatile memory such as a flash memory that holds the program 38 and various kinds of data. The storage is, for example, a large-capacity storage device such as a hard disk drive (HDD) or a solid state drive (SSD), and stores an operating system (OS), various kinds of data, and the like. The storage unit 34 may be configured as an external apparatus connected to the information processing device 30.

Variations occur in the waveform output from the plurality of measurement electrodes 51 included in the microelectrode array 50. For the toxicity evaluation, it is necessary to use a waveform close to an ideal waveform. The ideal waveform is, for example, a waveform representing a healthy state (for example, a state without a disease such as arrhythmia) obtained by the past measurement. From among the plurality of measurement electrodes 51, the measurement electrode 51 that outputs a waveform with the highest degree of similarity to the ideal waveform is selected as the target electrode for the toxicity evaluation. Such selection of the target electrode is also referred to as selection of a golden channel (hereinafter, referred to as GC).

The toxicity evaluation is performed by evaluating a change in waveform (for example, an extension of a QT interval) caused by adding a drug candidate compound to the culture solution of the well 42.

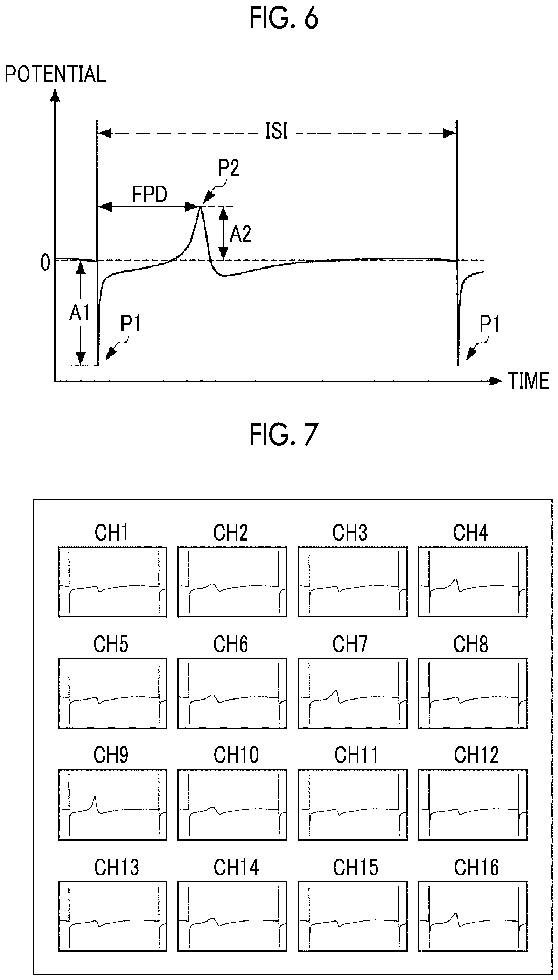

Fig. 6 shows an example of a myocardial waveform. The myocardial waveform shown in Fig. 6 is a measurement waveform of the extracellular potential. In Fig. 6, FPD is the above-mentioned extracellular potential duration, and ISI is the interspike interval. A1 is an amplitude of a first peak P1, and A2 is an amplitude of a second peak P2. These values vary depending on a state of the cell or various factors in measurement.

The information processing device 30 evaluates the degree of similarity to the ideal waveform using the FPD, the ISI, the maximum potential of P1, the minimum potential of P1, the value of A2, and the overall shape of the waveform as evaluation criteria.

Fig. 7 shows an example of waveforms output from the 16 measurement electrodes 51 included in the microelectrode array 50. Basically, among a plurality of waveforms obtained from the microelectrode array 50, the measurement electrode 51 from which a waveform closest to the ideal waveform is obtained is selected as the GC.

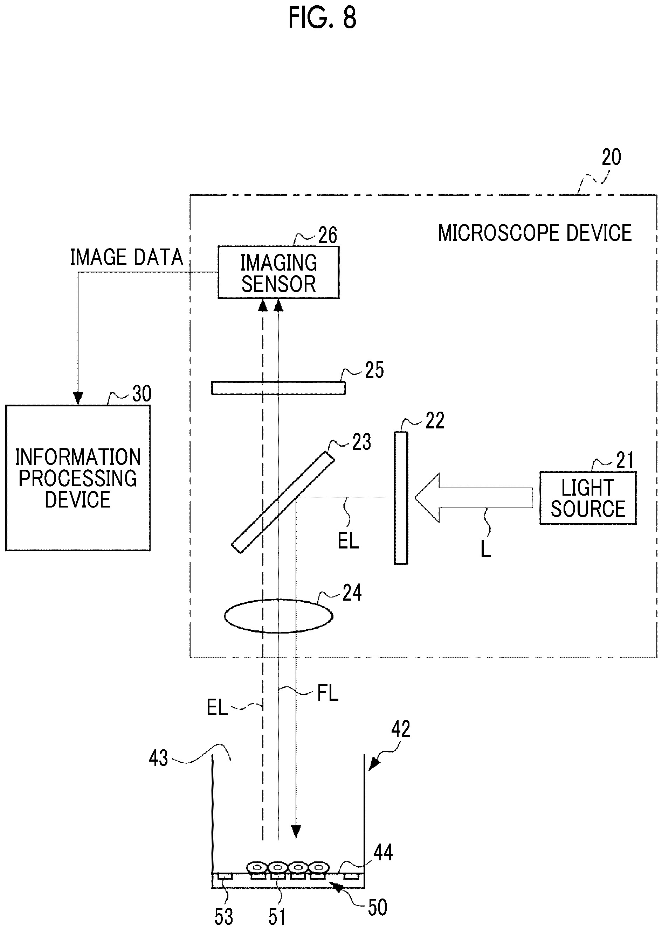

Fig. 8 schematically shows an example of a configuration of the microscope device 20. The cell group in the well 42 is observed by the microscope device 20 in a state in which the cells are stained with a fluorescent dye after the culture solution is removed from the well 42. For example, as the fluorescent dye, Hoechst (registered trademark), which is a nuclear staining dye for staining a nucleus of the cell, and Calcein, which is a living cell dye for staining the entire cell, are used.

The microscope device 20 is an epi-illumination microscope that images the measurement electrode 51 and the cells from a surface side of the measurement electrode 51 to which the cells adheres. A transmission microscope that captures an image of transmitted light of light applied to a back surface side of the measurement electrode 51 cannot image the cells on the measurement electrode 51, so that, in the present embodiment, the microscope device 20 is used as an epi-illumination microscope.

The microscope device 20 is an epifluorescence microscope configured to include a light source 21, an excitation filter 22, a dichroic mirror 23, an objective lens 24, an absorption filter 25, and an imaging sensor 26. The light source 21 is, for example, a mercury lamp, and emits light L having wavelengths from ultraviolet to near-infrared. The excitation filter 22 is a bandpass filter that extracts light having a wavelength required for excitation of a fluorescent substance (hereinafter, referred to as excitation light EL) from the light L emitted from the light source 21.

The dichroic mirror 23 reflects the excitation light EL and transmits fluorescence FL. The excitation light EL reflected by the dichroic mirror 23 passes through the objective lens 24 and is emitted from the opening 43 toward the bottom surface 44 of the well 42 to which the stained cell group is attached. The cell group is irradiated with the excitation light EL, thereby emitting the fluorescence FL. The fluorescence FL emitted from the cell group is transmitted through the objective lens 24 and the dichroic mirror 23 and is incident into the absorption filter 25.

The absorption filter 25 transmits the fluorescence FL and absorbs unnecessary scattered light other than the fluorescence FL. The imaging sensor 26 captures an image of the fluorescence FL transmitted through the absorption filter 25 to generate and output image data.

In the present embodiment, in order to image the electrodes including the reference electrode 53 and the measurement electrode 51 in addition to the cell group, the dichroic mirror 23 and the absorption filter 25 are configured to transmit a part of the excitation light EL reflected by the electrodes. That is, a region of the cells and a region of the electrodes on the bottom surface 44 of the well 42 can be specified based on the image data output from the imaging sensor 26.

In addition, the microscope device 20 is provided with a moving mechanism (not shown) that sequentially changes the wells 42 to be imaged, and the wells 42 in the MEA plate 40 are sequentially imaged.

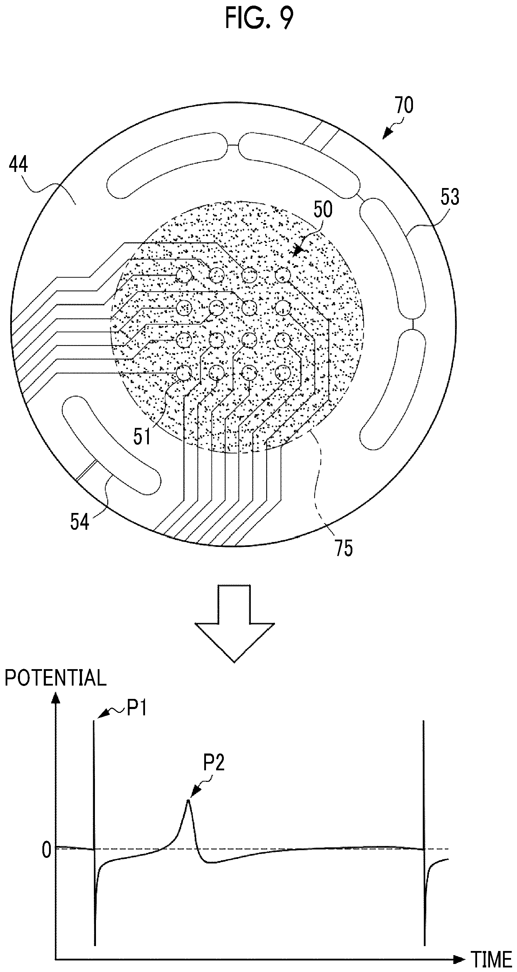

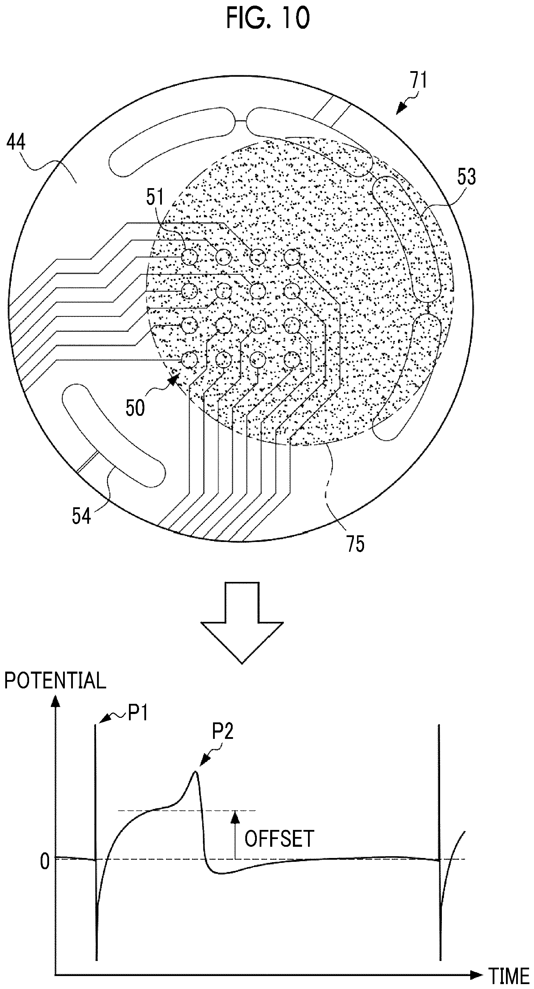

Figs. 9 and 10 show an example of a relationship between the seeding state of the cell group and the measurement waveform. An image 70 shown in Fig. 9 is an example of an image represented by image data obtained by the microscope device 20 in a case in which a cell group 75 is appropriately seeded on the bottom surface 44 of the well 42 in a state in which a waveform can be measured. That is, in Fig. 9, the cell group 75 is seeded so as to cover the microelectrode array 50 and not to be in contact with the reference electrode 53. In this case, a waveform close to the ideal waveform can be measured from the measurement electrode 51.

An image 71 shown in Fig. 10 is an example of an image represented by image data obtained by the microscope device 20 in a case in which the cell group 75 is not appropriately seeded on the bottom surface 44 of the well 42 in a state in which a waveform can be measured. In Fig. 10, the cell group 75 covers the microelectrode array 50 but is in contact with the reference electrode 53. That is, a plurality of cells are present on the reference electrode 53. In this case, an offset may occur in the waveform measured by the measurement electrode 51. In a case in which the offset becomes large, the second peak P2 may disappear and the FPD may not be measured. The present applicant has found that, ideally, the potential during the extracellular potential duration between the first peak P1 and the second peak P2 has to be a value close to the reference potential (0 V), but in a case in which cells are present on the reference electrode 53, an offset occurs due to a change in the reference potential.

Even in a case in which the measurement electrode 51 from which the waveform shown in Fig. 10 is measured is selected as the GC, the waveform has an offset and is not a waveform obtained by appropriate measurement, so that the waveform is not suitable for the toxicity evaluation. In the present embodiment, the GC is selected in consideration of the seeding state of the cells.

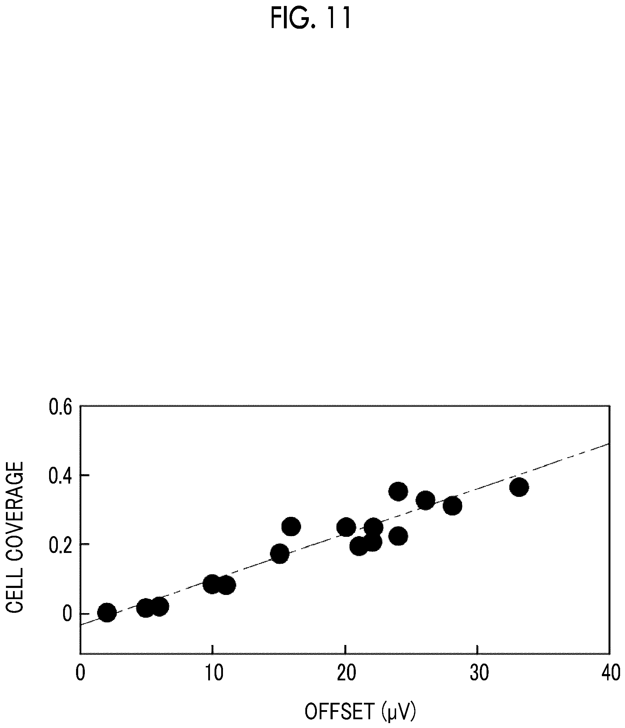

Fig. 11 shows a relationship between a cell coverage on the reference electrode 53 and the offset. The cell coverage is a value obtained by dividing an area of the cells on the reference electrode 53 by an area of the reference electrode 53. Fig. 11 shows that there is a strong correlation between the cell coverage and the offset, and that the offset increases as the cell coverage increases. For example, in a case in which the cell coverage is 0.05 or less, the waveform can be measured normally.

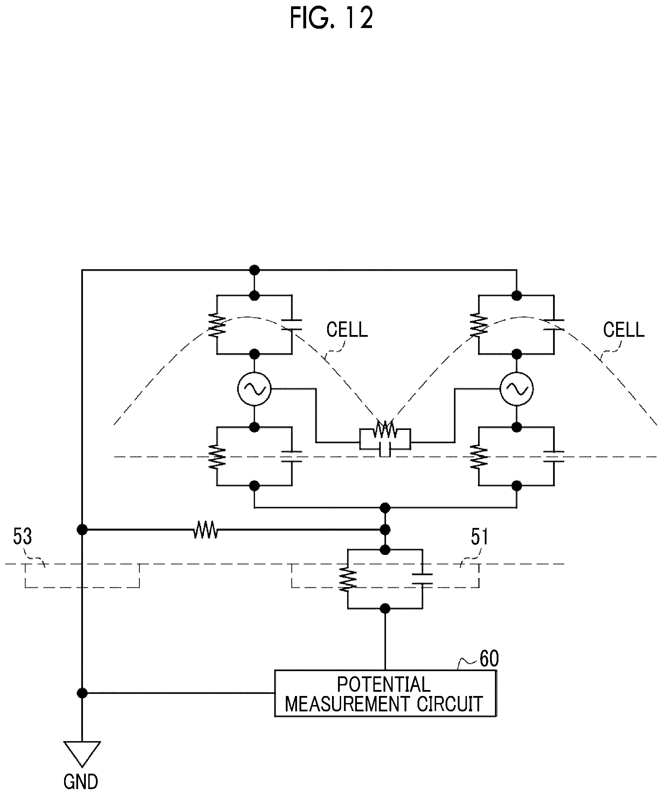

Fig. 12 is a diagram showing an example of an equivalent circuit of a model composed of the measurement electrode 51 and cells during waveform measurement. Fig. 12 shows a case in which two cells are present in a state of being bonded to the measurement electrode 51. As shown in Fig. 12, the equivalent circuit is represented by a resistance and a capacitance. The resistance and the capacitance of each part in the equivalent circuit are determined by factors such as an adhesion state between the cells and the measurement electrode 51, a thickness of a cell membrane, an expression level of an ion channel, a degree of gap junction between cells, and a composition of the culture medium. That is, these factors affect the measurement waveform.

In addition, in a case in which cells are present on the reference electrode 53 to which the ground potential is applied as the reference potential as described above, the reference potential changes, an offset occurs in the measurement waveform as described above.

In a case in which the cells are myocardial cells, the cell group synchronously exhibits a single behavior and acts, so that, in a case in which the waveform measurement is performed by the measurement electrode 51, a change in the reference potential inevitably occurs in the reference electrode 53 at the same time. Therefore, the influence on the measurement waveform due to the presence of the cells on the reference electrode 53 is large. On the other hand, in a case in which the cells are cells that act asynchronously like a nerve cell, the reference electrode 53 having a considerably large area is used, so that the potential change on the reference electrode 53 is averaged. Therefore, the influence on the measurement waveform due to the presence of the cells on the reference electrode 53 is smaller than in a case in which the cells are myocardial cells.

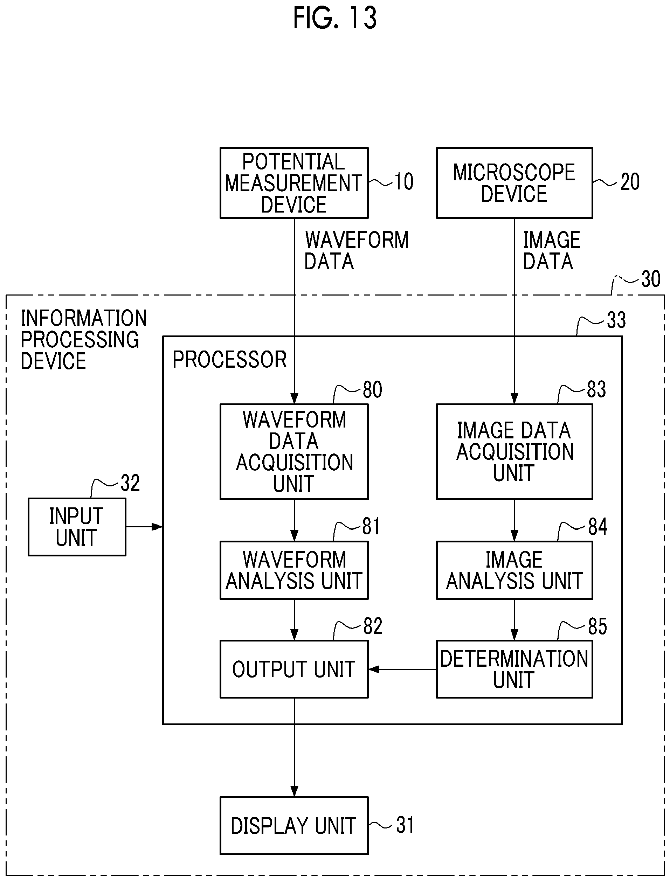

Fig. 13 shows an example of a functional configuration of the information processing device 30. As for the functions shown in Fig. 13, the information processing device 30 realizes various functions by the processor 33 executing processing based on the program 38 (see Fig. 5). These various functions may be realized by hardware.

The processor 33 functions as a waveform data acquisition unit 80, a waveform analysis unit 81, an output unit 82, an image data acquisition unit 83, an image analysis unit 84, and a determination unit 85. The waveform data acquisition unit 80 acquires a plurality of the waveform data output from the potential measurement device 10. The output unit 82 displays the waveform, the analysis result of the waveform data, and the like on the display unit 31.

The image data acquisition unit 83 acquires the image data from the microscope device 20. The image analysis unit 84 calculates the above-described cell coverage based on the image data. The determination unit 85 determines whether the seeding state of the cells is appropriate based on the cell coverage calculated by the image analysis unit 84. The output unit 82 displays the determination result of the seeding state determined by the determination unit 85 on the display unit 31. For example, the output unit 82 displays the determination result of the seeding state for each well 42 on the display unit 31.

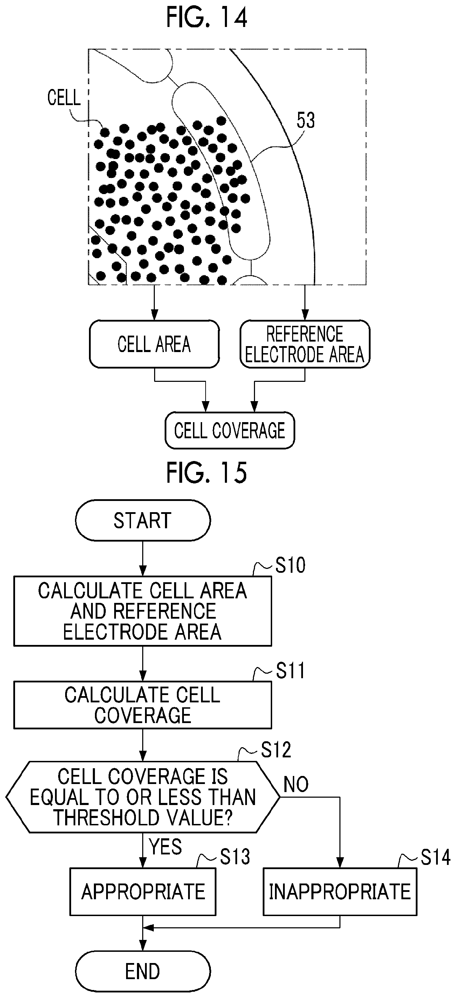

Fig. 14 conceptually shows an example of image analysis by the image analysis unit 84. As shown in Fig. 14, the image analysis unit 84 specifies a region of the cells and a region of the reference electrode 53 based on the image data, and calculates an area of the cells (hereinafter, referred to as a cell area) on the reference electrode 53 and an area of the reference electrode 53 (hereinafter, referred to as a reference electrode area). Then, the image analysis unit 84 calculates the cell coverage by dividing the cell area by the reference electrode area.

Fig. 15 shows an example of processing by the image analysis unit 84 and the determination unit 85. The image analysis unit 84 calculates the cell area and the reference electrode area based on the image data as described above (step S10), and calculates the cell coverage based on the calculated cell area and reference electrode area (step S11). The determination unit 85 determines whether the cell coverage is equal to or less than a threshold value (step S12). The threshold value is, for example, 0.05. In a case in which the cell coverage is equal to or less than the threshold value (step S12: YES), the determination unit 85 determines that the seeding state of the cells is appropriate (step S13). In a case in which the cell coverage is not equal to or less than the threshold value (step S12: NO), the determination unit 85 determines that the seeding state of the cells is inappropriate (step S14).

In a case in which the determination unit 85 determines that the seeding state of the cells is inappropriate, the output unit 82 may issue a warning to a user by displaying a message or the like on the display unit 31.

In a case in which the cell coverage is not equal to or less than the threshold value, the determination unit 85 may perform a process of estimating the influence (for example, the offset amount of the potential during the extracellular potential duration) on the measurement waveform based on the cell coverage. In this case, the output unit 82 displays the estimated value estimated by the determination unit 85, on the display unit 31. The user can determine whether the waveform is acceptable for the toxicity evaluation based on the estimated value.

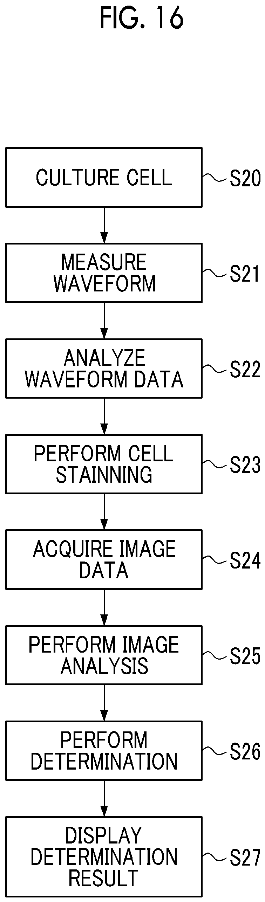

Fig. 16 shows an example of an evaluation flow by the evaluation system 2. First, the myocardial cells are seeded by dropping a cell suspension into each well 42 of the MEA plate 40, and then the cells are cultured by injecting a culture solution into each well 42 to create a cell group (myocardial sheet) (step S20). The cells are cultured for a period of about 1 week by a culture device. Next, the MEA plate 40 is set in the potential measurement device 10, and the myocardial waveform is measured (step S21). The plurality of waveform data acquired by the potential measurement device 10 are output to the information processing device 30, and the waveform data is analyzed by the information processing device 30 (step S22). In step S21, a drug candidate compound is added to the culture solution. In step S22, a change in the waveform before and after the addition of the drug candidate compound is analyzed.

Next, the MEA plate 40 is taken out from the potential measurement device 10, and the cell group is stained (step S23). After the cell group is stained, a plurality of image data are acquired by sequentially imaging the wells 42 of the MEA plate 40 using the microscope device 20 (step S24). The plurality of image data acquired by the microscope device 20 are output to the information processing device 30, and image analysis is performed on each piece of image data by the information processing device 30 (step S25). In step S25, the calculation of the cell coverage is performed. Then, the information processing device 30 determines the seeding state of the cells based on the calculated cell coverage (step S26), and displays the determination result on the display unit 31 (step S27).

As described above, according to the technology of the present disclosure, the reliability of the waveform (that is, whether the waveform is obtained by appropriate measurement) can be grasped based on the determination result of the seeding state, and the toxicity evaluation can be performed using the waveform with high reliability, thereby improving the evaluation accuracy of the toxicity evaluation.

### [Modification Example]

Hereinafter, various modification examples of the above-described embodiment will be described.

In the above-described embodiment, the image analysis unit 84 calculates the cell coverage of the reference electrode 53, but in a case in which the cell group is stimulated, a cell coverage of the stimulation electrode 54 may be calculated instead of or in addition to the reference electrode 53. This is because, in a case in which the stimulation electrode 54 is covered with cells, the stimulus applied from the stimulation electrode 54 to the cells is reduced below a desired intensity, which may affect the waveform.

In addition, in the above-described embodiment, the seeding state of the cells is determined based on the cell coverage of the reference electrode 53, but it may be determined whether the seeding state is appropriate based on other factors that are considered to affect the measurement waveform. For example, it may be determined whether the seeding state is appropriate by analyzing a state of the cells on the measurement electrode 51, such as the adhesiveness between the measurement electrode 51 and the cells, the cell density on the measurement electrode 51, and the variation in the cells on the measurement electrode 51.

The adhesiveness between the measurement electrode 51 and the cells can be evaluated by obtaining a thickness of the cells, a flatness of the cells, a distance between the measurement electrode 51 and the cells, and the like. The adhesiveness between the measurement electrode 51 and the cells can be accurately evaluated by using a confocal microscope, which is a type of epifluorescence microscope, as the microscope device 20. The confocal microscope can generate a three-dimensional image by acquiring images from a plurality of focal planes. The seeding state is more appropriate as the adhesiveness between the measurement electrode 51 and the cells increases. The adhesiveness between the measurement electrode 51 and the cells can also be determined by imaging the cells stained with a fluorescent dye for a factor related to the adhesiveness.

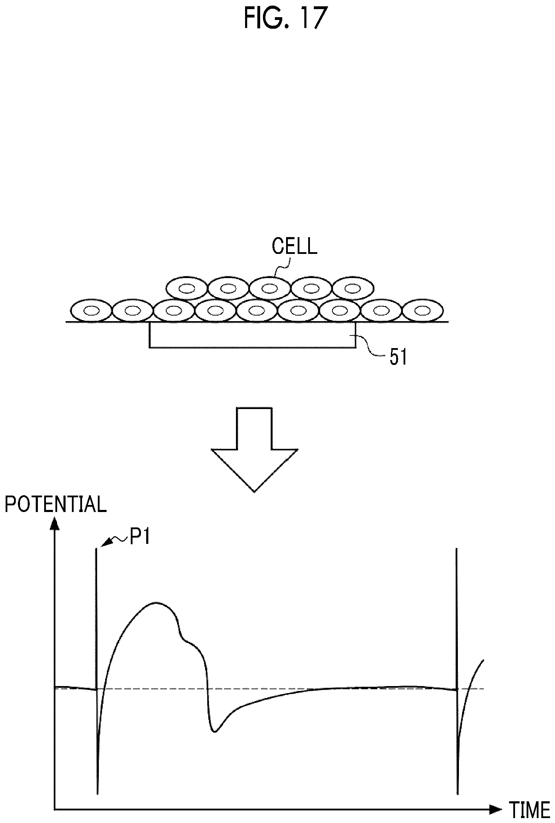

The cell density on the measurement electrode 51 increases as cells are laminated on the measurement electrode 51. For example, as shown in Fig. 17, in a case in which cells are laminated on the measurement electrode 51, a capacitor is laminated on the measurement electrode 51. As a result, in the measurement waveform, the second peak P2 disappears due to a rise in potential after the first peak P1, which may make it difficult to measure the FPD. The cell density on the measurement electrode 51 can be accurately evaluated by using a confocal microscope as the microscope device 20. It is preferable that the cell density on the measurement electrode 51 is within an appropriate range. Ideally, the cell density is preferably a value at which the cells are not laminated on the measurement electrode 51.

The variation in the cells on the measurement electrode 51 affects the expression level of the ion channel. It is preferable that the variation in the cells on the measurement electrode 51 is within an appropriate range.

In addition, since the waveform changes depending on the maturity of the cells, the maturity of the cells may be analyzed from the image data. The maturity of the cells can be determined using the cell morphology or fluorescence labeling. In a case in which the fluorescence labeling is used, a factor related to the maturity of the cells need only be stained with a fluorescent dye. For example, in a case in which the maturity of the cells is high, the cell morphology becomes oriented (that is, the cell elongates in a specific direction). In this way, the maturity of the cells can be determined by the orientation.

In addition, in the above-described embodiment, the microscope device 20 is used as an epifluorescence microscope, but a reflective type microscope can also be used instead of the epifluorescence microscope. In a case in which a reflective type microscope is used, the cell morphology is imaged without staining the cells with a fluorescent dye. In a case in which a reflective type microscope is used as the microscope device 20, the bottom surface 44 of the well 42 is illuminated with illumination light from obliquely upward, and an image of reflected light from the cells, the reference electrode 53, and the like is captured to generate image data. Further, instead of the epifluorescence microscope, a phase contrast microscope may be used to image the cell morphology without staining the cells with a fluorescent dye. The reflective type microscope and the phase contrast microscope can image the cell morphology without staining the cells, that is, non-destructively.

In addition, a model that is trained through machine learning may be used to acquire information equivalent to information obtained in a case in which the cells are imaged with an epifluorescence microscope, from the image data obtained by imaging the cells non-destructively using the reflective type microscope, the phase contrast microscope, or the like. For example, the model that is trained through machine learning is a deep learning model such as a convolutional neural network. The model that is trained through machine learning is a learning model that has been trained through machine learning using the image data obtained by imaging the cells non-destructively as input data and the image data obtained by imaging the cells with an epifluorescence microscope as correct answer data.

In addition, in the above-described embodiment, the imaging is performed by the microscope device 20 after the waveform measurement is performed by the potential measurement device 10, but the imaging may be performed by the microscope device 20 before the waveform measurement is performed by the potential measurement device 10. In a case in which a reflective type microscope is used as the microscope device 20, it is not necessary to stain the cells with a fluorescent dye, so that the imaging can be performed before the waveform measurement is performed. In addition, even though an epifluorescence microscope is used as the microscope device 20, in a case in which a fluorescent dye that does not affect the waveform measurement is used, it is also possible to perform the imaging before performing the waveform measurement.

In addition, in the above-described embodiment, the potential measurement device 10 and the microscope device 20 are configured as separate devices, but the microscope device 20 may be incorporated into the potential measurement device 10.

In addition, in the above embodiment, the potential measurement device 10 and the microscope device 20 are connected to one information processing device 30, but the potential measurement device 10 and the microscope device 20 may be connected to separate information processing devices.

In addition, in the above embodiment, the evaluation system 2 is configured to include the potential measurement device 10, the microscope device 20, and the information processing device 30, but the evaluation system according to the technology of the present disclosure need only include the microscope device and the information processing device.

In the above-described embodiment, the myocardial cells are used as the cells, but it is also possible to use cells such as nerve cells instead of the myocardial cells.

In the above-described embodiment, for example, a hardware structure of a processing unit that executes various kinds of processing, such as the waveform data acquisition unit 80, the waveform analysis unit 81, the output unit 82, the image data acquisition unit 83, the image analysis unit 84, and the determination unit 85, is various processors as shown below.

Various processors include a CPU, a programmable logic device (PLD), a dedicated electric circuit, and the like. As is well known, the CPU is a general-purpose processor that executes software (program) and functions as various processing units. The PLD is a processor whose circuit configuration can be changed after manufacturing, such as a field programmable gate array (FPGA). The dedicated electric circuit is a processor that has a dedicated circuit configuration designed to perform a specific process, such as an application specific integrated circuit (ASIC).

One processing unit may be configured of one of these various processors, or may be configured of a combination of two or more processors of the same type or different types (for example, a combination of a plurality of FPGAs or a combination of a CPU and an FPGA). In addition, a plurality of processing units may be constituted by one processor. As an example in which the plurality of processing units are constituted by one processor, first, one processor is constituted by a combination of one or more CPUs and software and this processor functions as the plurality of processing units. Second, as typified by a system on chip (SoC) or the like, a processor that realizes the functions of the entire system including the plurality of processing units by using one IC chip is used. As described above, the various processing units are configured using one or more of the various processors as a hardware structure.

More specifically, the hardware structure of these various processors is an electric circuit (circuitry) in which circuit elements such as semiconductor elements are combined.

The present disclosure is not limited to the embodiment described above and may adopt various configurations without departing from the spirit of the present disclosure. In addition to the program, the present disclosure extends to a computer-readable storage medium that stores the program in a non-temporary manner.

It is possible to grasp the following technologies by the above description.

### [Appendix 1]

An evaluation system that evaluates a seeding state of cells seeded in a cell culture vessel having at least one electrode disposed on a bottom surface, the evaluation system comprising:
a microscope device that generates image data by imaging the electrode and the cells from a surface side of the electrode of the cell culture vessel; and
an information processing device that analyzes the image data and determines whether the seeding state is appropriate based on an analysis result.

### [Appendix 2]

The evaluation system according to Appendix 1,
in which the microscope device is an epifluorescence microscope that generates the image data by irradiating the cells stained with a fluorescent dye with excitation light and capturing an image of fluorescence emitted from the cells.

### [Appendix 3]

The evaluation system according to Appendix 2,
in which the microscope device generates the image data for specifying a region of the cells and a region of the electrode by capturing an image of the excitation light reflected by the electrode in addition to the fluorescence.

### [Appendix 4]

The evaluation system according to Appendix 1,
in which the microscope device generates the image data for specifying a region of the cells and a region of the electrode by irradiating the cells and the electrode with illumination light and capturing an image of reflected light from the cells and the electrode.

### [Appendix 5]

The evaluation system according to Appendix 3 or 4,
in which the electrode includes a measurement electrode for measuring a potential and at least one of a reference electrode to which a reference potential is applied or a stimulation electrode for applying a stimulus to the cells.

### [Appendix 6]

The evaluation system according to Appendix 5,
in which the cells are smaller than a size of the measurement electrode, and a plurality of the cells are seeded on the measurement electrode.

### [Appendix 7]

The evaluation system according to Appendix 6,
in which the plurality of seeded cells exhibit a single behavior.

### [Appendix 8]

The evaluation system according to Appendix 7,
in which the cells are myocardial cells.

### [Appendix 9]

The evaluation system according to any one of Appendices 5 to 8,
in which the information processing device calculates a cell coverage on the reference electrode or on the stimulation electrode by analyzing the image data, and determines that the seeding state is appropriate in a case in which the calculated cell coverage is equal to or less than a threshold value.

### [Appendix 10]

The evaluation system according to Appendix 9,
in which, in a case in which the cell coverage is not equal to or less than the threshold value, the information processing device estimates an influence on a measurement waveform based on the cell coverage.

### [Appendix 11]

The evaluation system according to any one of Appendices 5 to 8,
in which the information processing device analyzes a state of the cells on the measurement electrode based on the image data.

### [Appendix 12]

The evaluation system according to any one of Appendices 1 to 11,
in which the information processing device issues a warning in a case in which the seeding state is not appropriate.

### [Appendix 13]

An information processing device that evaluates a seeding state of cells seeded in a cell culture vessel having at least one electrode disposed on a bottom surface, the information processing device comprising:
a processor,
in which the processor
   acquires image data generated by imaging the electrode and the cells from a surface side of the electrode of the cell culture vessel with a microscope device, and
   analyzes the image data and determines whether the seeding state is appropriate based on an analysis result.

### [Appendix 14]

An information processing method of evaluating a seeding state of cells seeded in a cell culture vessel having at least one electrode disposed on a bottom surface, the information processing method comprising:
acquiring image data generated by imaging the electrode and the cells from a surface side of the electrode of the cell culture vessel with a microscope device; and
analyzing the image data and determining whether the seeding state is appropriate based on an analysis result.

## Claims

1. An evaluation system that evaluates a seeding state of cells seeded in a cell culture vessel having at least one electrode disposed on a bottom surface, the evaluation system comprising:
a microscope device that generates image data by imaging the electrode and the cells from a surface side of the electrode of the cell culture vessel; and
an information processing device that analyzes the image data and determines whether the seeding state is appropriate based on an analysis result.

2. The evaluation system according to claim 1,
wherein the microscope device is an epifluorescence microscope that generates the image data by irradiating the cells stained with a fluorescent dye with excitation light and capturing an image of fluorescence emitted from the cells.

3. The evaluation system according to claim 2,
wherein the microscope device generates the image data for specifying a region of the cells and a region of the electrode by capturing an image of the excitation light reflected by the electrode in addition to the fluorescence.

4. The evaluation system according to claim 1,
wherein the microscope device generates the image data for specifying a region of the cells and a region of the electrode by irradiating the cells and the electrode with illumination light and capturing an image of reflected light from the cells and the electrode.

5. The evaluation system according to claim 3 or 4,
wherein the electrode includes a measurement electrode for measuring a potential and at least one of a reference electrode to which a reference potential is applied or a stimulation electrode for applying a stimulus to the cells.

6. The evaluation system according to claim 5,
wherein the cells are smaller than a size of the measurement electrode, and a plurality of the cells are seeded on the measurement electrode.

7. The evaluation system according to claim 6,
wherein the plurality of seeded cells exhibit a single behavior.

8. The evaluation system according to claim 7,
wherein the cells are myocardial cells.

9. The evaluation system according to claim 5,
wherein the information processing device calculates a cell coverage on the reference electrode or on the stimulation electrode by analyzing the image data, and determines that the seeding state is appropriate in a case in which the calculated cell coverage is equal to or less than a threshold value.

10. The evaluation system according to claim 9,
wherein, in a case in which the cell coverage is not equal to or less than the threshold value, the information processing device estimates an influence on a measurement waveform based on the cell coverage.

11. The evaluation system according to claim 5,
wherein the information processing device analyzes a state of the cells on the measurement electrode based on the image data.

12. The evaluation system according to claim 1,
wherein the information processing device issues a warning in a case in which the seeding state is not appropriate.

13. An information processing device that evaluates a seeding state of cells seeded in a cell culture vessel having at least one electrode disposed on a bottom surface, the information processing device comprising:
a processor,
wherein the processor
acquires image data generated by imaging the electrode and the cells from a surface side of the electrode of the cell culture vessel with a microscope device, and
analyzes the image data and determines whether the seeding state is appropriate based on an analysis result.

14. An information processing method of evaluating a seeding state of cells seeded in a cell culture vessel having at least one electrode disposed on a bottom surface, the information processing method comprising:
acquiring image data generated by imaging the electrode and the cells from a surface side of the electrode of the cell culture vessel with a microscope device; and
analyzing the image data and determining whether the seeding state is appropriate based on an analysis result.
